# EUROPEAN PATENT SPECIFICATION

(11) **EP 3 517 599 B1**
(45) Date of publication and mention of the grant of the patent: **10.11.2021**
(21) Application number: 17853492.1
(22) Date of filing: 25.09.2017
(51) Int. Cl.: A61Q 19/00, C12J 1/00, A61K 8/98, A61K 8/36, A61Q 19/02

(54) **COMPOSITION FOR IMPROVING SKIN CONDITION INCLUDING FERMENTED PEARL PRODUCT**
ZUSAMMENSETZUNG MIT EINEM FERMENTIERTEN PERLENPRODUKT ZUR VERBESSERUNG DES HAUTZUSTANDES
COMPOSITION COMPRENANT UN PRODUIT DE PERLE FERMENTÉ POUR ATTÉNUER UNE AFFECTION CUTANÉE

(30) Priority: 23.09.2016 KR 20160122312; 06.09.2017 KR 20170113624
(43) Date of publication of application: 31.07.2019
(73) Proprietor: Sempio Foods Company, Seoul 04557 (KR)
(72) Inventor: BAEK, Eun Jong, Incheon 21920 (KR); JEONG, Gang Hee, Cheongju-si Chungcheongbuk-do 28166 (KR); JEON, Jong Eun, Seoul 04362 (KR); CHOI, Yong Ho, Cheongju-si Chungcheongbuk-do 28660 (KR); HURH, Byung Serk, Sejong 30101 (KR); RYU, Jeoung Jin, Seongnam-si Gyeonggi-do 13486 (KR); KANG, Seung Hyun, Seongnam-si Gyeonggi-do 13486 (KR); NAM, Jin Ju, Seongnam-si Gyeonggi-do 13486 (KR); NHO, Youn Hwa, Seongnam-si Gyeonggi-do 13486 (KR); KIM, Youn Joon, Seongnam-si Gyeonggi-do 13486 (KR); CHEON, Ji Eun, Cheongju-si Chungcheongbuk-do 28166 (KR); EUN, Byong Wook, Anyang-si Gyeonggi-do 13985 (KR)
(74) Representative: Schmitz, Joseph
(86) International application number: PCT/KR2017/010555
(87) International publication number: WO 2018/056778

(56) References cited:
- CN-A- 1 686 082
- CN-A- 104 523 522
- DE-A1- 10 215 342
- JP-A- 2007 167 056
- JP-A- 2007 217 345
- JP-A- 2007 217 345
- JP-B2- 5 317 301
- KR-A- 20120 066 721
- KR-B1- 101 321 611

## Description

### [Technical Field]

The present invention relates to the use of a cosmetic composition for improving skin condition comprising a fermented pearl product as defined in the appended claims.

More specifically, the present invention provides the use of a composition for improving skin condition which comprises a fermented pearl product having remarkably increased ingredients such as amino acids, polyphenol, and organic calcium content, and has a remarkably enhanced skin condition-improving effect such as activity of inhibiting melanin production, and promoting differentiation and exfoliation of keratinocytes, as compared with conventional pearl extracts.

### [Background Art]

Pearls contain a large amount of calcium which is calcium carbonate secreted by epithelial cells of the oyster mantle. In a case where a foreign matter enters an oyster, conchiolin protein allows the foreign matter to be made into several layers of small particles of aragonite which is a crystal of calcium carbonate, and therefore, a layer of pearl is formed. Thus, main ingredients of pearls consist of 90% to 95% of calcium carbonate (CaCO₃), about 5% of protein (conchiolin), and 20 or more other types of minerals and amino acids which are various physiologically active substances. Due to these constituent ingredients, pearls are widely used as supplementary health foods or skin cosmetic materials. It is known that in a case where pearl powders are ingested as a calcium source or pearls are used for skin-aging prevention and whitening effects, the skin is maintained in a slightly acidic state to increase skin immunity.

However, pearl powders have a disadvantage that due to not being soluble in water, useful ingredients are difficult to be absorbed in the body. In the prior art, in order to improve this disadvantage, a pearl extract, which is obtained by treating pearl powders with acetic acid or the like so that the pearl powders are converted into a water-soluble form, is used.

However, conventional pearl extracts are only in the form of water-soluble pearl powders in which calcium carbonate of pearls is converted into an ionized state. There has been no report on a composition which is prepared so that useful ingredients and other additional ingredients of pearls can act on the skin in multiple ways to exhibit a better effect in improving skin condition. See in this context e.g. the disclosure provided in CN104523522 A.

Meanwhile, factors that determine human skin color include melanin, thickness of the stratum corneum, peripheral blood vessel and hemoglobin, carotene, and the like. Among these, melanin is the most important factor in determining skin color. The number of melanin-producing cells that produce melanin pigment is constant irrespective of race or skin color, but difference in skin color is due to difference in degree of melaninization, size and number of melanosomes, and distribution state thereof, melanosome degradation in keratinocytes, and the like. In particular, white skin has many melanosomes at stage I and II, and black skin has many melanocytes at stage III and IV (Lee, Seungheon et al., "Skin Barrier", Ryo Moon Gak.P.Co, 2010). Since the protein pre-melanosomal protein 17 (PMEL17) is involved in maturation of melanin, melanin production efficacy can be utilized as a main factor that indicates whitening.

One of the most important functions of the skin is its function as a barrier. Skin, which is directly exposed to an external environment, is the most important primary line of defense which prevents loss of body fluids and protects a body from harmful environments. The stratum corneum, which is located at the outermost part of the skin and is in direct contact with the outside, plays the most important role in skin barrier function although the stratum corneum is in a non-nucleated state. In other words, the stratum corneum is a skin protective shield which protects the skin from external stimuli and prevents water from being evaporated from the skin, and plays a key role in skin health.

Keratinocytes are produced in the basal layer and then matured and keratinized. As a result, the keratinocytes have a flattened cell shape and are exfoliated after loss of intracellular organelles, production of fibrous proteins, dehydration, and cell membrane thickening. This process causes the epidermis to undergo a process of proliferation, differentiation, and exfoliation, and is most important for maintenance of skin regeneration homeostasis. In particular, in a differentiation process of keratinocytes, as the keratinocytes produced in the basal layer of the epidermis reach the granular layer, the keratinocytes begin to lose intracellular organs, and calcium ions are introduced into the keratinocytes to activate transglutaminase so that irreversible binding of proteins, which constitute the cornified cell envelope, such as involucrin, loricrin, and cornifin is induced. An epidermal differentiation process which normally occurs through this process plays an important role in skin barrier function.

As a final step in the differentiation process, the keratinocytes at the uppermost layer are exfoliated and detached from the skin. A normal exfoliation process of the stratum corneum keeps the skin radiant and healthy. The exfoliation process is also meaningful in removing harmful microorganisms, viruses, and the like introduced from the outside along with keratin. In this process, various proteases are involved. Such proteases are normally activated in a weakly acidic state. Kallikrein-related peptidases 5 and 7 play an important role as modulators for detachment of the skin stratum corneum. Therefore, normalization of differentiation and exfoliation in the epidermis layer keeps the outermost layer of the skin healthy, and causes a skin barrier function to be smoothly performed, thereby lowering a water evaporation amount.

### [Disclosure]

### [Technical Problem]

A fermented pearl product obtained through a method of adding pearl powders to fermented apple vinegar and performing after-fermentation exhibits an excellent skin condition-improving effect.

A composition comprising the fermented pearl product to be used in the present invention contains, at high concentrations, active ingredients of fermented apple vinegar and calcium that originates from pearl powders and has a high absorption rate into the body. Upon application to the skin, due to multiple action of these ingredients, the composition promotes inhibition of melanin production and differentiation of keratinocytes, and exhibits a superior effect for skin condition improvement such as skin whitening and skin moisturizing.

Accordingly, an object of the present invention is to provide the use of a composition for improving skin condition comprising a fermented pearl product.

### [Technical Solution]

According to an aspect of the present invention, there is provided the use of a composition for improving skin condition, comprising a fermented pearl product prepared by adding, to fermented apple vinegar, pearl powders in an amount ranging from 0.05% to 20% (w/v) and performing after-fermentation.

In the composition, the fermented apple vinegar may have a polyphenol content of 50 to 1,000 ppm and an amino acid content of 50 to 500 ppm, and may have a fresh flavor property.

Apples used in the fermented apple vinegar may be, but are not limited to, at least one type selected from cultivars such as early variety, mid variety, and late variety.

The fermented apple vinegar used in the composition for improving skin condition of the present invention is mixed with pearl powders and subjected to after-fermentation. Thus, not only the fermented apple vinegar effectively extracts useful ingredients such as calcium, conchiolin, and amino acids of pearls, but also useful ingredients of the fermented apple vinegar itself act in multiple ways together with the useful ingredients of pearls, so that the composition can provide a remarkably enhanced skin condition-improving effect as compared with conventional pearl extracts.

According to a preferred embodiment of the present invention, the fermented pearl product has a total polyphenol content of 100 to 1,000 ppm, a total amino acid content of 300 to 1,000 ppm, and a total calcium content of 1,000 to 20,000 ppm.

In the present invention, the after-fermentation is performed at 20°C to 40°C for 3 to 7 days.

In a preferred embodiment of the present invention, pearl powders used in the present invention may be added in an amount ranging from about 0.01% to 30% (w/v), more particularly about 0.03% to 25% (w/v), and even more particularly about 0.05% to 20% (w/v), based on a volume of the fermented apple vinegar.

In the composition for improving skin of the present invention, a fermented pearl product in which pearl powders are contained within the above-mentioned range is effective to provide an optimal skin condition-improving effect, together with amino acid and polyphenol ingredients of the fermented vinegar. In a case where the pearl powders are used in excess of the above-mentioned range, an unnecessary excess amount of pearl powders is used and causes undissolved pearl powders to exist, resulting in decreased efficiency in term of economy.

Calcium is not only a constituent ingredient for growth of skeletal tissue, bones, and teeth, but is also an important mineral in the body's physiological regulation functions, such as activation of various enzymes in the body, regulation of neuronal excitement, muscle contraction and relaxation, regular heart rate, and blood coagulation. However, calcium is not well absorbed into the body. The fermented pearl product obtained through the after-fermentation step contains various minerals and amino acids, in particular, ionized high-concentration calcium ions, which originate from the pearl powders. Therefore, the fermented pearl product finally obtained in the present invention can be applied as a cosmetic raw material from the viewpoint that useful ingredients of the fermented apple vinegar and a calcium ingredient are contained.

The improvement in skin condition caused by the fermented pearl product of the present invention is an improvement in skin regeneration, skin whitening, skin moisturizing, skin elasticity, skin aging, or wrinkle.

The present inventors have identified, through the following Experimental Examples 1 to 4, that in a case where amino acids and polyphenol exist together with calcium, an excellent effect in improving skin condition is exhibited even at a small amount.

Specifically, the fermented pearl product of the present invention not only contains high-concentration calcium which originates from pearl powders and has a high absorption rate into the body, but also contains amino acid and polyphenol ingredients which originate from the fermented apple vinegar. These ingredients act in multiple ways on the skin, so that a remarkably excellent skin condition-improving effect is exhibited as compared with a case where a pearl extract and fermented vinegar are used alone at an equal amount. This effect is a multiple synergistic effect caused by co-application of calcium, amino acids, and polyphenol to the skin.

As illustrated in FIG. 2, in a case of being administered to pigment cells, the fermented pearl product of the present invention induces inhibitory activity against melanin production which is superior to fermented vinegar and a pearl extract. In addition, as illustrated in FIGS. 3A and 3B, the fermented pearl product of the present invention remarkably increases expression levels of IVL and LOR as compared with fermented vinegar and a pearl extract, thereby exhibiting an excellent skin turnover effect. As illustrated in FIGS. 3C, 3D, and 4, the fermented pearl product of the present invention increases expression levels of KLK-5 and KLK-7, thereby providing an improved skin barrier-strengthening effect and a skin moisturizing function as compared with the pearl extract.

Through these results, it was possible to identify that the fermented pearl product used has an excellent activity of inhibiting melanin production, and promoting differentiation of keratinocyte and removal of the stratum corneum, as compared with a pearl extract, and exhibits an enhanced skin condition-improving effect as compared with the pearl extract.

In an embodiment of the present invention, the composition used for improving skin condition of the present invention may be a cosmetic composition, comprising (a) a cosmetically effective amount of the fermented pearl product as described above; and (b) a cosmetically acceptable carrier.

As used herein, the term "cosmetically effective amount" means an amount sufficient to achieve skin-improving efficacy of the composition of the present invention as described above.

The cosmetic composition used in the present invention may be prepared in any of formulations which are conventionally prepared in the art, and may be formulated into, but is not limited to, a solution, a suspension, an emulsion, a paste, a gel, a cream, a lotion, a powder, a soap, a surfactant-containing cleanser, oil, powder foundation, emulsion foundation, wax foundation, a spray, or the like. More specifically, the cosmetic composition of the present invention may be prepared in a formulation which is a soft lotion, a nutritional lotion, a nutritional cream, a massage cream, an essence, an eye cream, a cleansing cream, a cleansing foam, a cleansing water, a pack, a spray, or a powder.

In a case where the formulation of the present invention is a paste, a cream, or a gel, animal oil, vegetable oil, wax, paraffin, starch, tragacanth, cellulose derivative, polyethylene glycol, silicone, bentonite, silica, talc, or zinc oxide may be used as a carrier ingredient.

In a case where the formulation of the present invention is a powder or a spray, lactose, talc, silica, aluminum hydroxide, calcium silicate, or polyamide powder may be used as a carrier ingredient. In particular, in a case of the spray, a propellant such as chlorofluorohydrocarbon, propane/butane, or dimethyl ether may be additionally contained.

In a case where the formulation of the present invention is a solution or an emulsion, a solvent, a solubilizer, or an emulsifier is used as a carrier ingredient, and examples thereof include water, ethanol, isopropanol, ethyl carbonate, ethyl acetate, benzyl alcohol, benzyl benzoate, propylene glycol, 1,3-butyl glycol oil, glycerol aliphatic ester, polyethylene glycol, and fatty acid ester of sorbitan.

In a case where the formulation of the present invention is a suspension, a liquid diluent such as water, ethanol, or propylene glycol, a suspending agent such as ethoxylated isostearyl alcohol, polyoxyethylene sorbitol ester, and polyoxyethylene sorbitan ester, microcrystalline cellulose, aluminum metahydroxide, bentonite, agar, tragacanth, or the like may be used as a carrier ingredient.

In a case where the formulation of the present invention is a surfactant-containing cleanser, aliphatic alcohol sulfate, aliphatic alcohol ether sulfate, sulfosuccinic acid monoester, isethionate, imidazolinium derivative, methyltaurate, sarcosinate, fatty acid amide ether sulfate, alkylamidobetaine, aliphatic alcohol, fatty acid glyceride, fatty acid diethanolamide, vegetable oil, lanolin derivative, ethoxylated glycerol fatty acid ester, or the like may be used as a carrier ingredient.

Ingredients contained in the cosmetic composition of the present invention include, in addition to the fermented pearl product as an active ingredient and the carrier ingredient, ingredients commonly used in cosmetic compositions, for example, conventional adjuvants such as antioxidants, stabilizers, solubilizers, vitamins, pigments, and fragrances.

### [Advantageous Effects]

Features and advantages of the present invention are summarized as follows:
(a) The fermented pearl product not only has high contents of ingredients such as amino acids and polyphenol as compared with common vinegar, but also has a remarkably high content of calcium ion which is ionized and easily absorbed into the body.
(b) The composition comprising the fermented pearl product of the present invention has activity of inhibiting melanin production, and promoting differentiation and exfoliation of keratinocytes, and thus can remarkably improve skin condition.
(c) In addition, the fermented pearl product of the present invention provides a remarkably enhanced skin condition-improving effect as compared with conventional pearl extracts.
(d) Therefore, the composition of the present invention can be simply and effectively applied to a cosmetic composition.

### [Description of Drawings]

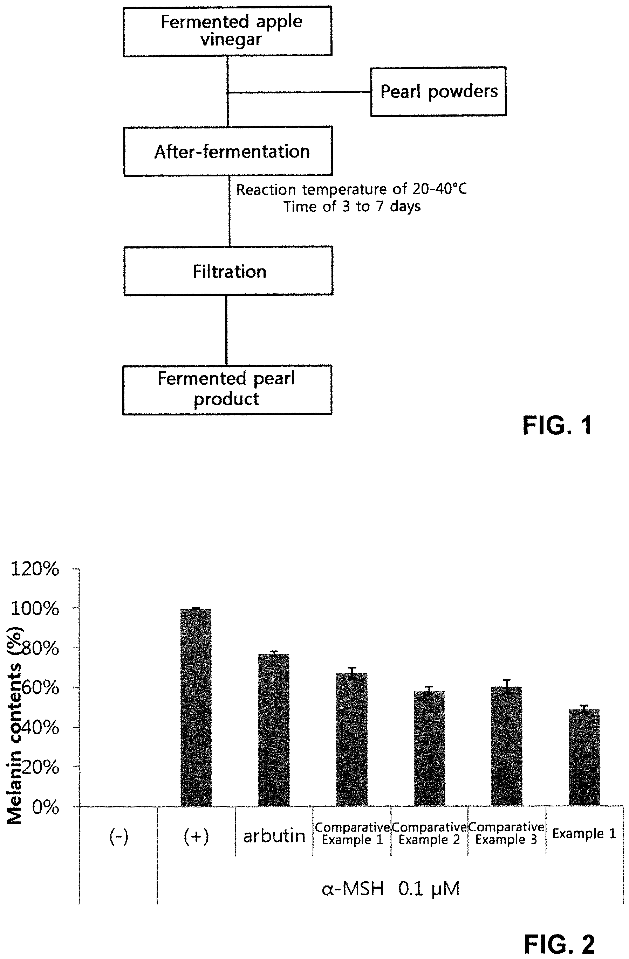

FIG. 1 illustrates a process flow diagram for preparing the fermented pearl product of the present invention.
FIG. 2 illustrates results obtained by measuring melanin contents in mouse pigment cells (B16 melanoma cells) which had been treated with each of spirit vinegar, fermented apple vinegar, pearl extract, and fermented pearl product.

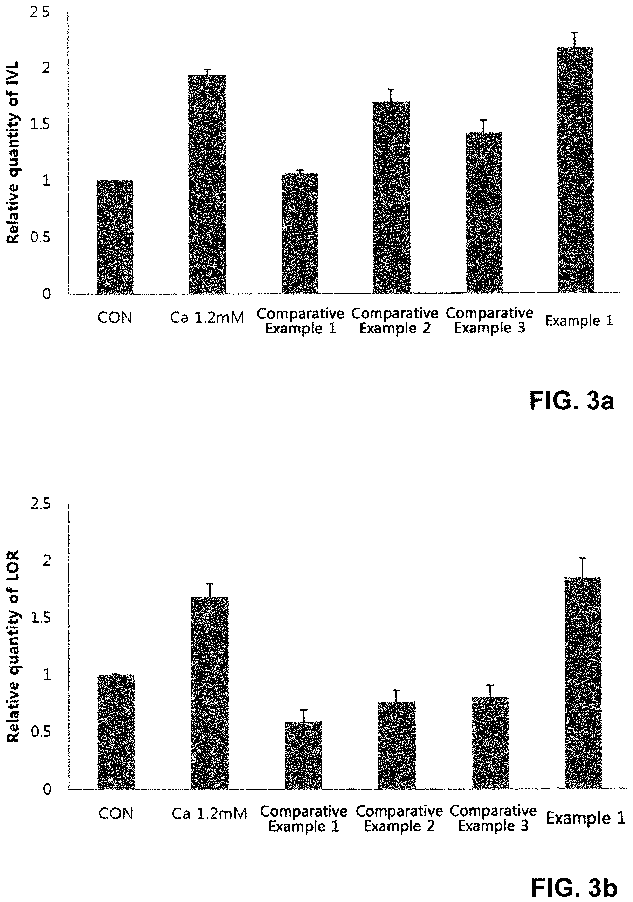

FIGS. 3A to 3D illustrate results which show expression levels of respective genes (IVL, LOR, KLK-5, and KLK-7) identified by real-time PCR. For a negative control, ^{∗}: p<0.05, ^{∗∗}: p<0.01.

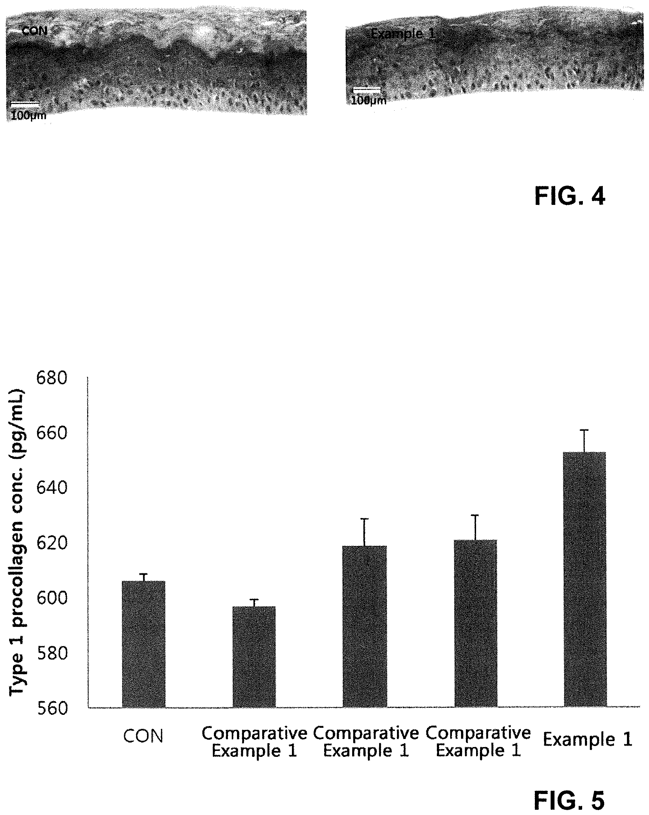

FIG. 4 illustrates results of staining in the artificial skin from which an exfoliation level of the stratum corneum is observed. CON means a control.
FIG. 5 illustrates results which show an expression level of type 1 procollagen gene identified by real-time PCR. For a negative control, ^{∗}: p<0.05, ^{∗∗}: p<0.01.

### [Modes of the Invention]

Hereinafter, the present invention will be described in more detail with reference to examples.

### [Examples]

### Example 1: Preparation of fermented pearl product

10% (w/v) of pearl powders were added to a fermented apple vinegar stock solution and then after-fermentation was performed at 25°C for 3 to 7 days. Upon completion of the fermentation, a "fermented pearl product" that contains ionized calcium at a high concentration was prepared through microfiltration (pore size: 0.1 µL).

### Comparative Example 1: Spirit vinegar

Spirit vinegar diluted to 1% by weight was used as Comparative Example 1.

### Comparative Example 2: Fermented apple vinegar

Fermented apple vinegar diluted to 1% by weight was used as Comparative Example 2.

### Comparative Example 3: Preparation of pearl extract

For the pearl extract of Comparative Example 3, pearl powders were placed in a suitable container, an appropriate amount of water was added therein so that the pearl powders absorbed the water, and then extra water was added therein. At this time, washing filtration was performed once or twice. To 1 g of the washed pearl solids was slowly added a 10% acetic acid (CH₃COOH) aqueous solution so that a final weight became 90 g. The mixture was stirred at 350 rpm at room temperature. When no carbon dioxide was generated, it was judged that the reaction was completed, and supernatant was recovered. The supernatant was neutralized with diluted alkali, and then water was added so that a final product became 100 g. Then, filtration was performed to prepare 1% by weight of a pearl extract, which was used in the following experimental examples.

### Experimental Example 1: Whitening - Evaluation for inhibition of melanin production

Inhibitory effects on melanin production caused by treatment with the compositions of Comparative Examples 1, 2, and 3, and Example 1 were measured using mouse pigment cells (B16 melanoma cells, ATCC, CRL-6475™). Such inhibitory effects were compared with inhibitory effects on melanin production caused by arbutin (100 µg/ml) which is known to inhibit melanin production.

Mouse pigment cells (B16 F10) were suspended in DMEM containing 10% fetal bovine serum (FBS) and inoculated into a 6-well plate at 1 × 10⁵ cells per well. Culture was performed until the cells adhered to a well bottom. In order to induce melanin production, treatment with 0.1 µM α-MSH was performed, and culture was performed for 3 days after addition of the sample. A concentration at which treatment with a substance was performed was set to 100 µg/ml. Arbutin was used as a positive control in an experiment for melanin production capability. The cultured cells were washed with phosphate-buffered saline (PBS) and recovered with trypsin. The recovered cells were counted with a hematocytometer, collected so that the same cell number at 1 × 10⁶ cells/ml was obtained for each treatment group, and centrifuged at 1,000 rpm for 10 minutes to obtain cells. The recovered cells were dried at 60°C for 1 hour, and then 400 µl of a 1 M NaOH solution containing 10% DMSO was added to obtain intracellular melanin. An inhibition rate for melanin production was evaluated by measuring an absorbance at 490 nm of the melanin solution using a microplate reader.

As can be identified in FIG. 2, melanin contents in the mouse pigment cells were decreased by the treatment with the compositions of Comparative Examples 1, 2, and 3, and Example 1. In particular, the fermented pearl product of Example 1 exhibited the best inhibitory activity against melanin production.

### Experimental Example 2: Analysis of gene expression in keratinocyte cell line

### 2-1. Culture of HaCaT cells, human skin keratinocyte cell line

Keratinocytes HaCaT (CLS, 300493), a constitutive cell line of human skin, were cultured using a DMEM culture solution that contains 10% FBS and antibiotics. A 75T-flask was used as a container for culture, and culture was performed in an incubator at 37°C to which 5% CO₂ is supplied. The culture solution was replaced every 3 to 4 days and subculture was performed when the cells were excessively grown. The HaCaT cells were dispensed into a 6-well plate (5 × 10⁵/well), cultured for 24 hours using DMEM, and then washed with phosphate buffered saline (PBS). To each well was added each of 1.2 mM calcium chloride, and the compositions of Comparative Example 1, Comparative Example 2, and Comparative Example 3, and Example 1, diluted in a Ca-free DMEM medium containing no FBS so as to be 1% by weight. After 24 hours, cDNA was synthesized and PCR was performed to evaluate gene expression levels.

### 2-2. Test for effects on keratinocyte differentiation and expression of exfoliating factors using real-time PCR

The synthesized cDNA was subjected to real-time PCR using primers and an SYBR green master mixture which is a cyanine dye, so that gene expression levels were finally evaluated. Comparison for expression of genes was made with a relative value obtained through calibrated quantification of the internal control gene β-actin according to the Ct (threshold cycle) method, and primer sequences used are shown in Table 1 below.

**[Table 1]**

| **Primer name** | **Sequence (5'→3')** | | SEQ ID NO |
|---|---|---|---|
| IVL | Forward | TGAAACAGCCAACTCCACTG | SEQ ID NO: 1 |
| | Reverse | GGAGCTCCAACAGTTGCTCT | SEQ ID NO: 2 |
| LOR | Forward | AATAGATCCCCCAGGGTACCA | SEQ ID NO: 3 |
| | Reverse | CGGTGCCCCTGGAAAAC | SEQ ID NO: 4 |
| KLK-5 | Forward | GCCACACTGCAGGAAGAAA | SEQ ID NO: 5 |
| | Reverse | GGATTTGACCCCCTGGAA | SEQ ID NO: 6 |
| KLK-7 | Forward | GCATCCCCGACTCCAAGAA | SEQ ID NO: 7 |
| | Reverse | CAGGGTACCTCTGCACACCAA | SEQ ID NO: 8 |
| β-Actin | Forward | GGCCATCTCTTGCTCGAAGT | SEQ ID NO: 9 |
| | Reverse | GACACCTTCAACACCCCAGC | SEQ ID NO: 10 |

As can be seen in FIGS. 3A and 3B, it was identified that treatment with a 1% fermented pearl product of Example 1 results in significantly increased expression levels of involucrin (IVL), loricrin (LOR), kallikrein-related peptidase-5 (KLK-5), and KLK-7, as compared with the other treatment groups. More specifically, increased expression of IVL and LOR means that epidermal keratinocyte differentiation is promoted and skin turnover is caused to occur normally. An expression level of IVL was significantly increased in the fermented pearl product of Example 1 as compared with the fermented apple vinegar Comparative Example 2 and the pearl extract of Comparative Example 3. In particular, in a case of an expression level of LOR, the fermented pearl product exhibits a remarkable synergistic effect which cannot be easily predicted from the other treatment groups. Thus, it was identified that the fermented pearl product of Example 1 can induce a superior skin turnover effect.

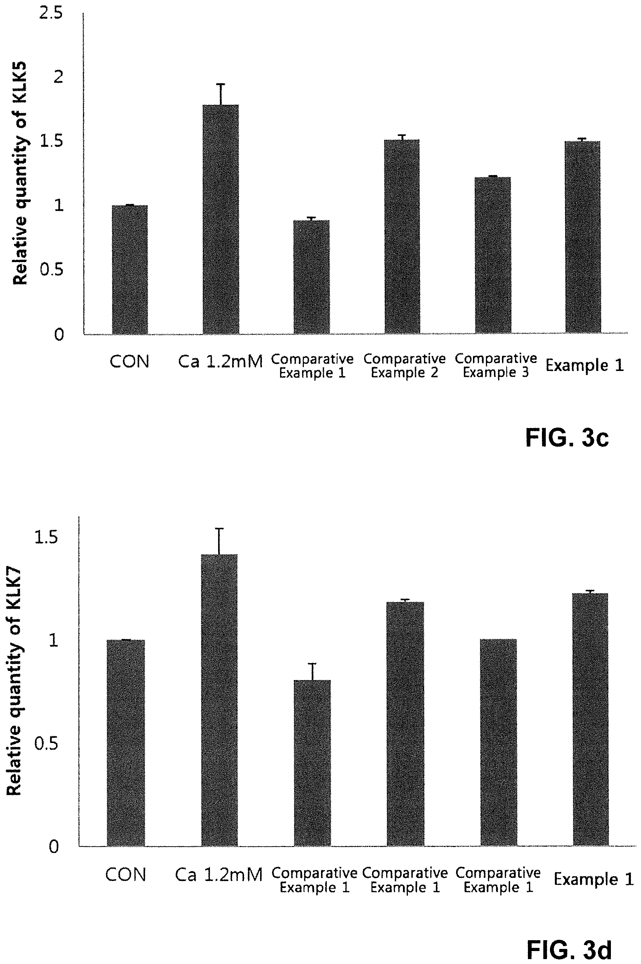

In addition, an increase in KLK-5 and KLK-7 means that the outermost stratum corneum is normally exfoliated. As illustrated in FIGS. 3C and 3D, it was identified that the fermented pearl product of Example 1 promotes an epidermal differentiation and exfoliation capability as compared with the pearl extract, thereby strengthening a skin barrier and exhibiting an excellent effect in skin moisturizing function.

### Experimental Example 3: Histological observation of skin

### 3-1. Artificial skin culture

Levels of epidermal differentiation and expression of exfoliation factors caused by the fermented pearl product of Example 1 were identified in the reconstructed human epidermis (RHE), a three-dimensional cultured skin model which is reproduced to resemble human skin. Upon arrival of RHE, culture was performed in a growth medium for 24 hours for stabilization, and then treatment with 1% of each of 1.2 mM calcium chloride and the fermented pearl product of Example 1 was performed.

### 3-2: Experiment on stratum corneum exfoliation effect using H&E staining

After 2 days of culture, extracted skin tissue was fixed in a 10% formalin solution. Then, paraffin sections were cut and tissue immunostaining was performed.

As a result, as illustrated in FIG. 4, it is identifiable that the stratum corneum was normally exfoliated over time and no thickened stratum corneum was formed in a group treated with the fermented pearl product of Example 1, as compared with a control CON. These results indicate that the fermented pearl product of Example 1 effectively induces epidermal turnover in a skin-histological manner and has an effect of improving a skin barrier function.

### Experimental Example 4: Analysis of gene expression in fibroblast cell line

### 4-1. Culture of Hs68 cells, human skin fibroblast cell line

Fibroblast cell line Hs68 (ATCC, CRL-1635™), a constitutive cell line of human skin, was cultured in a DMEM culture solution that contains 10% FBS and antibiotics. A 75T-flask was used as a container for culture, and culture was performed in an incubator at 37°C to which 5% CO₂ is supplied. The culture solution was replaced every 3 to 4 days and subculture was performed when the cells were excessively grown. The Hs68 cells were dispensed into a 6-well plate (4 × 105/well), cultured for 24 hours using DMEM, and then washed with phosphate buffered saline (PBS). To each well was added each of the compositions of Comparative Example 1, Comparative Example 2, and Comparative Example 3, and Example 1, diluted in a Ca-free DMEM medium containing no FBS so as to be 1% by weight. After 24 hours, cDNA was synthesized and PCR was performed to evaluate gene expression levels.

### 4-2. Identification of procollagen production in fibroblasts using real-time PCR

The synthesized cDNA was subjected to real-time PCR using primers and an SYBR green master mixture which is a cyanine dye, so that an expression level of procollagen gene was finally evaluated. Comparison for expression of gene was made with a relative value obtained through calibrated quantification of the internal control gene β-actin according to the Cₜ (threshold cycle) method, and primer sequences used are shown in Table 2 below.

**[Table 2]**

| **Primer name** | **Sequence (5'→3')** | | SEQ ID NO |
|---|---|---|---|
| Type 1 procollagen | Forward | CTCGAGGTGGACACCACCCT | SEQ ID NO: 11 |
| | Reverse | CAGCTGGATGGCCACATCGG | SEQ ID NO: 12 |
| β-Actin | Forward | GGCCATCTCTTGCTCGAAGT | SEQ ID NO: 9 |
| | Reverse | GACACCTTCAACACCCCAGC | SEQ ID NO: 10 |

As can be seen in FIG. 5, it was identified that treatment with a 1% fermented pearl product of Example 1 results in a significantly increased expression level of type 1 procollagen, as compared with the other treatment groups. More specifically, increased expression of type 1 procollagen which is a precursor of type 1 collagen mainly produced in fibroblasts and is a major causative agent in determining a thickness of the skin dermal layer, means inhibition of wrinkle formation. From the above results, it can be seen that the fermented pearl product of Example 1 can increase production of type 1 collagen, which acts as a support in the dermis, to induce an effect capable of inhibiting wrinkle and sagging phenomena which are typical aspects of skin aging.

### Experimental Example 5: Analysis of ingredients of fermented pearl product

In this experimental example, changes in active ingredients of the fermented pearl product prepared in Example 1 were identified (Table 3).

**[Table 3]**

| Item | Comparative Example 1 (spirit vinegar) | Comparative Example 2 (fermented apple vinegar) | Example 1 (fermented pearl product) |
|---|---|---|---|
| Total amino acids (ppm) | 170 | 372 | 441 |
| Calcium (ppm) | 3 | 92 | 11,148 |
| Total polyphenol (ppm) | 0 | 130 | 125 |

## Claims

1. Use of a composition in improving skin condition, comprising: a fermented pearl product prepared by adding pearl powders in an amount ranging from 0.05% to 20% (w/v) and performing after-fermentation with fermented apple vinegar at 20°C to 40°C for 3 to 7 days, wherein the skin condition to be improved is an improvement in skin regeneration, skin whitening, skin moisturizing, skin elasticity, skin aging, or wrinkle.

2. The use according to claim 1, wherein the fermented pearl product has a total polyphenol content of 100 to 1,000 ppm, a total amino acid content of 300 to 1,000 ppm, and a total calcium content of 1,000 to 20,000 ppm.

3. The use according to claim 1, which inhibits melanin production.

4. The use according to claim 1, which promotes differentiation of keratinocytes.

5. The use according to claim 1, which promotes removal of the stratum corneum.

## Patentansprüche

1. Verwendung einer Zusammensetzung zur Verbesserung des Hautzustands, umfassend:
ein fermentiertes Perlenprodukt, hergestellt durch Zugabe von Perlenpulvern in einer Menge im Bereich von 0,05% bis 20% (Gew./Vol.) und Durchführung einer Nachfermentation mit fermentiertem Apfelessig bei 20 °C bis 40 °C während 3 bis 7 Tagen, **dadurch gekennzeichnet dass** die Verbesserung des Hautzustands eine Verbesserung der Hautregeneration, der Hautaufhellung, der Hautbefeuchtung, der Hautelastizität, der Hautalterung oder der Falten ist.

2. Die Verwendung nach Anspruch 1, wobei das fermentierte Perlenprodukt einen Gesamtpolyphenolgehalt von 100 bis 1.000 ppm, einen Gesamtaminosäuregehalt von 300 bis 1.000 ppm und einen Gesamtkalziumgehalt von 1.000 bis 20.000 ppm aufweist.

3. Die Verwendung nach Anspruch 1, die die Melaninproduktion hemmt.

4. Die Verwendung nach Anspruch 1, die die Differenzierung von Keratinozyten fördert.

5. Die Verwendung nach Anspruch 1, die die Entfernung des Stratum corneum fördert.

## Revendications

1. Utilisation d'une composition dans l'amélioration de l'état de la peau, comprenant:
un produit à base de perle fermentée préparé en ajoutant des poudres de perle en une quantité allant de 0,05 % à 20 % (p/v) et en réalisant une post-fermentation avec du vinaigre de pomme fermenté à 20 °C à 40 °C pendant 3 à 7 jours, dans laquelle l'état de la peau à améliorer est une amélioration de la régénération de la peau, du blanchiment de la peau, humidification de la peau, élasticité de la peau, vieillissement de la peau ou des rides.

2. L'utilisation selon la revendication 1, dans laquelle le produit à base de perle fermentée comprend une teneur totale en polyphénols de 100 à 1 000 ppm, une teneur totale en acides aminés de 300 à 1 000 ppm et une teneur totale en calcium de 1 000 à 20 000 ppm.

3. L'utilisation selon la revendication 1, qui inhibe la production de mélanine.

4. L'utilisation selon la revendication 1, qui favorise la différenciation des kératinocytes.

5. L'utilisation selon la revendication 1, qui favorise l'élimination de la couche cornée.


<110> SEMPIO FOODS COMPANY COSMAX, INC.
<120> Composition for Improving Skin Conditions Comprising Pearl Fermentation Products
<130> PCT5036749
<150> KR 10-2016-0122312
   <151> 2016-09-23
<150> KR 10-2017-0113624
   <151> 2017-09-06
<160> 12
<170> KopatentIn 2.0
<210> 1
   <211> 20
   <212> DNA
   <213> Artificial Sequence
<220>
   <223> IVL forward primer
<400> 1
   tgaaacagcc aactccactg 20
<210> 2
   <211> 20
   <212> DNA
   <213> Artificial Sequence
<220>
   <223> IVL reverse primer
<400> 2
   ggagctccaa cagttgctct 20
<210> 3
   <211> 21
   <212> DNA
   <213> Artificial Sequence
<220>
   <223> LOR forward primer
<400> 3
   aatagatccc ccagggtacc a 21
<210> 4
   <211> 17
   <212> DNA
   <213> Artificial Sequence
<220>
   <223> LOR reverse primer
<400> 4
   cggtgcccct ggaaaac 17
<210> 5
   <211> 19
   <212> DNA
   <213> Artificial Sequence
<220>
   <223> KLK-5 forward primer
<400> 5
   gccacactgc aggaagaaa 19
<210> 6
   <211> 18
   <212> DNA
   <213> Artificial Sequence
<220>
   <223> KLK-5 reverse primer
<400> 6
   ggatttgacc ccctggaa 18
<210> 7
   <211> 19
   <212> DNA
   <213> Artificial Sequence
<220>
   <223> KLK-7 forward primer
<400> 7
   gcatccccga ctccaagaa 19
<210> 8
   <211> 21
   <212> DNA
   <213> Artificial Sequence
<220>
   <223> KLK-7 reverse primer
<400> 8
   cagggtacct ctgcacacca a 21
<210> 9
   <211> 20
   <212> DNA
   <213> Artificial Sequence
<220>
   <223> Beta-actin forward primer
<400> 9
   ggccatctct tgctcgaagt 20
<210> 10
   <211> 20
   <212> DNA
   <213> Artificial Sequence
<220>
   <223> Beta-actin reverse primer
<400> 10
   gacaccttca acaccccagc 20
<210> 11
   <211> 20
   <212> DNA
   <213> Artificial Sequence
<220>
   <223> Type 1 procollagen forward primer
<400> 11
   ctcgaggtgg acaccaccct 20
<210> 12
   <211> 20
   <212> DNA
   <213> Artificial Sequence
<220>
   <223> Type 1 procollagen reverse primer
<400> 12
   cagctggatg gccacatcgg 20